Europäisches Patentamt

European Patent Office

Office européen des brevets

(19)

(11) Numéro de publication : **0 025 733**
**B1**

(12) **FASCICULE DE BREVET EUROPÉEN**

(45) Date de publication du fascicule du brevet :
25.07.84

(51) Int. Cl.³ : **C 06 B 25/04, C 06 B 21/00**

(21) Numéro de dépôt : **80401195.5**

(22) Date de dépôt : **20.08.80**

(54) **Procédé de purification du trinitro-2,4,6 toluène.**

(30) Priorité : **13.09.79 FR 7922912**

(43) Date de publication de la demande :
**25.03.81 Bulletin 81/12**

(45) Mention de la délivrance du brevet :
**25.07.84 Bulletin 84/30**

(84) Etats contractants désignés :
**FR**

(56) Documents cités :
**EP-A- 0 016 481**
**M. MEYER: "The Science of Explosives", 1943, page 258, T.Y. CROWELL COMPANY, NEW YORK (US), "Crystallization from sulphuric acid"**
**T.L. DAVIS: "The Chemistry of Powder and Explosives", 1943, page 145, J. WILEY & SONS, Inc., NEW YORK (US)**
**INDUSTRIAL AND ENGINEERING CHEMISTRY, vol. 42, 1 mars 1950, EASTON, PA. (US) M.L. KASTENS: "TNT into Phloroglucinol", pages 402-413**
**"Military Explosives", Technical Manual no. 9-1910, 14 avril 1955, "Departments of the Army and the Air Force", page 145, WASHINGTON, D.C. (US)**

(73) Titulaire : **SOCIETE NATIONALE DES POUDRES ET EXPLOSIFS**
**12, quai Henri IV**
**F-75181 Paris Cedex 04 (FR)**

(72) Inventeur : **Ousset André**
**2b, allée des Saules**
**F-84700 Sorgues (FR)**

Jouve, 18, rue St-Denis, 75001 Paris, France

# 0 025 733

## Description

L'invention concerne un procédé de purification du trinitrotoluène. On sait que le trinitrotoluène (encore dénommé T.N.T. ou tolite) est une matière puissamment explosive largement utilisée seule ou en mélanges dans les chargements explosifs militaires ou civils. On sait aussi que le trinitrotoluène est un intermédiaire de synthèse d'autres explosifs tel que le trinitrobenzène ou de produits chimiques utiles à l'industrie civile tel que le phloroglucinol ou trihydroxy-1,3,5 benzène.

L'un des principaux freins à l'utilisation du trinitrotoluène dans l'industrie chimique civile est la grande sensibilité de ce produit dans les conditions ordinairement rencontrées dans ce secteur d'activité. Il est sensible aux chocs et au frottement et, lorsqu'il est en masse, sa combustion peut se transformer en une détonation. Les poussières de T.N.T. sont en outre très sensibles aux effets de l'électricité statique et sont très toxiques puisque la teneur minimale permise est de 1,5 mg/m$^3$ dans l'atmosphère des locaux industriels. Dans les conditions habituelles, le T.N.T. est transporté et stocké sous forme de granulés ou de paillettes sèches, en vrac. D'après ce qui précède et du fait de l'augmentation du confinement dans les conteneurs utilisés, on comprend que les opérations de transport du T.N.T. sont entourées de précautions fort contraignantes. Une solution pour flegmatiser le T.N.T. consiste à lui conférer un taux d'humidité d'au moins 7 % en poids. On sait en effet qu'alors le T.N.T. n'est plus susceptible de détoner quand il est soumis à des sollicitations mécaniques ordinaires. Malheureusement la présence d'eau si elle permet d'atteindre un certain degré de sécurité, n'est pas du tout souhaitée par les utilisateurs et son élimination, compte tenu de l'augmentation notable de la sensibilité du T.N.T. en fonction de l'augmentation de la température, est délicate ce qui grève fortement le prix relativement faible dudit T.N.T.

Il a maintenant été trouvé des solutions à base de T.N.T., efficacement flegmatisées, n'affectant pas la réactivité ultérieure du T.N.T. et permettant d'obtenir un T.N.T. purifié sans recourir à une élévation de température dangereuse.

L'invention concerne donc un procédé de purification du trinitro-2,4,6 toluène caractérisé en ce qu'on forme une solution homogène comprenant de 7 à 30 % en poids de trinitro-2,4,6 toluène à purifier, de 50 à 80 % en poids d'acide sulfurique et de 10 à 28 % en poids d'anhydride sulfurique puis en ce qu'on provoque la séparation du trinitro-2,4,6 toluène et en ce qu'on recueille ce dernier à l'état solide sous une forme purifiée.

Il a en effet été trouvé que le T.N.T. qu'on peut récupérer d'une telle solution a un degré de pureté supérieur ou égal à celui du T.N.T. qu'on y a placé initialement. L'effet de purification est naturellement d'autant plus sensible que le T.N.T. initial est moins pur.

Selon une variante particulièrement préférée de l'invention la solution contient de 11 à 24 % de trinitro-2,4,6 toluène, de 55 à 72 % en poids d'acide sulfurique et de 11 à 23 % en poids d'anhydride sulfurique.

La qualité du T.N.T. utilisée dans le cadre de la présente invention n'est pas critique mais il est préférable qu'elle soit la meilleure possible. Il est connu d'une manière générale que le T.N.T. est d'autant plus sensible qu'il est moins pur. Il s'est avéré, dans le cadre de l'invention, qu'un T.N.T. ayant un point de fusion supérieur à 79 °C et de préférence supérieur à 79,4 °C convient particulièrement bien.

En pratique, les solutions selon l'invention sont réalisées en mélangeant le T.N.T. avec de l'oléum sulfurique, jusqu'à obtention d'une solution homogène, à température ordinaire. L'oléum sulfurique devra en pratique contenir de préférence de 15 à 40 % en poids, environ, d'anhydride sulfurique. Un oléum peu riche en SO$_3$ solubilise en effet peu de T.N.T. alors qu'un oléum très riche en SO$_3$ est plus difficile à réaliser et à utiliser. Un oléum comprenant entre 20 et 35 % en poids d'anhydride sulfurique est particulièrement préféré. La dissolution rapide du T.N.T. dans l'oléum peut se faire à température ambiante pour les solutions peu chargées ou bien à chaud, soit 40 °C environ, pour les solutions plus chargées, comprenant, par exemple, plus de 20 % de T.N.T. en poids.

Le degré de flegmatisation de ces solutions peut être considéré comme très satisfaisant et tout à fait inattendu. Si l'on pouvait en effet espérer, du fait de la bonne stabilité chimique du T.N.T., que les solutions seraient chimiquement stables tout au moins dans les conditions standards, en revanche il était pratiquement assuré pour l'homme de l'art qu'elles seraient explosibles. Ce préjugé tient à la fois à la sensibilité intrinsèque du T.N.T. et au caractère puissamment oxydant de l'oléum sulfurique. De plus la balance d'oxygène de l'oléum sulfurique est positive, ce qui compense la balance d'oxygène légèrement négative du T.N.T. et confère à ces solutions une balance d'oxygène plus proche de l'équilibre que ne l'est celle du T.N.T. pur.

De fait, il a été observé que ces solutions donnent des résultats se situant à la limite ou en deçà de la limite des principaux tests permettant d'apprécier l'explosibilité d'une composition. Ainsi les tests de Julius Peters de sensibilité au frottement où à l'impact ont donné des résultats négatifs dans les conditions les plus sévères prévues dans ces textes. Le test d'aptitude à la détonation derrière barrière (ou Card Gap Test) donne une non-détonation pour moins d'une carte. Le test du mouton de choc de 30 kg donne un résultat négatif même pour la hauteur de 4 m. Le test de combustion en gouttière aboutit à une non-inflammation. Enfin le test de chauffage externe indique que seul un confinement d'au plus 1 mm conduit à un résultat positif, et ce, encore, pour les solutions les plus chargées en T.N.T.

Cette flegmatisation poussée rend ces solutions particulièrement aptes à être transportées ou stockées comme matières certes corrosives, mais non explosives.

Selon une première variante préférée du procédé selon l'invention on sépare le trinitro-2,4,6 toluène par addition dans ladite solution homogène d'un fluide diminuant la solubilité du T.N.T. dans ladite solution. Selon une variante particulièrement préférée, le fluide est choisi dans le groupe constitué par l'eau et l'acide sulfurique faiblement concentré (moins de 30 % en poids d'H$_2$SO$_4$).

Selon une seconde variante préférée du procédé selon l'invention, qui peut être cumulée avec la précédente, on sépare le T.N.T. en abaissant la température de ladite solution jusqu'à précipitation du T.N.T.

Dans l'une et l'autre variante, on recueille le T.N.T. purifié par tout moyen habituel, par exemple, par filtration. La purification du T.N.T. se traduit par une augmentation sensible du point de fusion et de la stabilité sous vide.

Dans les exemples non limitatifs suivants ressortiront d'autres avantages liés à l'invention.

## Exemple 1

On a réalisé une solution selon l'invention constituée de 18,9 % en poids de T.N.T., 60,8 % en poids d'acide sulfurique et de 20,3 % en poids de trioxyde de soufre, SO$_3$. Pour ce faire, on a introduit 45 g de T.N.T. de point de fusion 80,45 °C dans 100 ml d'oléum sulfurique à 25 % de SO$_3$ et on a agité le mélange pendant une heure à température ambiante pour obtenir une solution limpide.

La densité de cette solution est de 1,82 et sa viscosité à 25 °C de 62,7 mPl. On a soumis des échantillons de cette solution à différents tests de conservation : 1) séjour d'un mois en étuve à 50 °C, 2) séjour d'un mois en étuve à 75 °C, 3) cycle thermique de 10 heures à 40 °C, refroidissement à 20 °C en deux heures, 10 heures à 20 °C, réchauffage à 40 °C en deux heures et ce, pendant dix cycles successifs. On a obtenu les résultats suivants consistant en une comparaison des qualités du T.N.T. avant et après le test.

| | T.N.T. initial | Test (1) à 50°C | Test (2) à 75°C | Cycle (3) |
|---|---|---|---|---|
| Point de fusion | 80,45°C | 80,54°C | 80,55°C | 80,49°C |
| S.S.V.* | 0,065 | 0,036 | 0,032 | 0,058 |

*stabilité sous vide : volume de gaz dégagé par un échantillon en 100 heures à 100 °C, en cm$^3$/g.

En outre le chromatogramme en phase gazeuse de l'échantillon ne subit aucune modification après le test. Sur le spectre infra-rouge des T.N.T. après les tests (1) et (2) on constate une atténuation d'un léger décrochement à 393 cm$^{-1}$, ce qui correspond à la disparition d'une trace d'impureté.

Ces tests montrent la bonne tenue au stockage des solutions. Le T.N.T. sortant amélioré de ces épreuves, cela indique que son degré de pureté a été amélioré.

## Exemples 2 à 5

On a réalisé trois solutions en vue de tester leur capacité à détoner.
— La première (EX2) est la même qu'à l'exemple 1.
— La seconde (EX3) comprend 13,4 % de T.N.T., 64,9 % d'H$_2$SO$_4$ et 21,7 % de SO$_3$ et a été préparé comme à l'exemple 1 mais en dissolvant le T.N.T. à raison de 30 g pour 100 ml d'oléum sulfurique à 25 % en poids de SO$_3$ (dont la masse volumique est 1,934 g/cm$^3$).
— La troisième (EX4) comprend 23,7 % en poids de T.N.T., 57,2 5 % en poids d'H$_2$SO$_4$ et 19,1 % en poids de SO$_3$ et a été préparé par dissolution de T.N.T. dans de l'oléum sulfurique à 25 % en poids de SO$_3$, à 40 °C, en une heure, à raison de 60 g de T.N.T. par 100 ml d'oléum. Le T.N.T. utilisé était de qualité T, c'est-à-dire présentait un point de fusion supérieur à 80,1 °C.

**0 025 733**

Les épreuves sont généralement effectuées à température ambiante. On a toutefois testé la composition correspondant à la solution EX4 à − 45 °C environ, car elle ne se présente plus sous forme d'un liquide mais sous la forme d'un solide. Cette composition sous cet état a été appelée EX5.

| | | EX2 | EX3 | EX4 | EX5 |
|---|---|---|---|---|---|
| Aptitude à la déto nation derrière barrière | | 1 carte | 1 carte | 1 carte | 1 carte |
| Sensibilité à l'impact Julius PETERS | | -- | 0% à 5 kgm | 0% à 5 kgm | - |
| Mouton de 30 kg | Résultat | > 4 m | > 4 m | > 4 m | > 4 m |
| | Observations | aucune réaction | aucune réaction | aucune réaction | aucune réaction |
| Sensibilité au frottement Julius PETERS | | - | - | - | 0% à 36 kgf |
| chauf- fage externe | 0 en mm | - | 1 mm | 1 mm | 1 mm |
| | $T_1$ en s | - | 21 s | 24 s | 23 s |
| | $T_2$ en s | - | - | 53 s | - |
| Combustion en gouttière | | - | non inflammation | non inflammation | non inflammation |

L'épreuve d'aptitude à la détonation derrière barrière consiste à rechercher la barrière, en cartes d'acétate de cellulose, nécessaire pour empêcher la transmission de la détonation entre une cartouche d'explosif initiatrice et une cartouche confinée d'explosif à éprouver.

L'explosif à tester contenu dans un tube à essais en verre pyrex diamètre 36,5/39,5 mm est introduit dans un tube acier, type A37, de 40,2 mm de diamètre intérieur, 4,05 mm d'épaisseur et de 200 mm de longueur. La résistance statique calculée moyenne du tube acier est d'environ 745 bars.

On prépare un tir en introduisant successivement dans un tube carton de diamètre 50 mm : un comprimé d'hexocire de diamètre 40 mm et masse 80 g, le tube d'acier contenant l'explosif à tester, un certain nombre de cartes d'acétate de cellulose et un relais excitateur d'hexocire de diamètre 40 mm et masse 320 g qui sera amorcé par un détonateur n° 8. Le tout est placé sur une plaque témoin en acier doux (150 × 150 × 10 mm).

La quantité de l'explosif nécessaire à l'épreuve, est de 1 titre. Les tirs ont lieu avec le montage vertical et suspendu.

On recherche par une série de tirs (pas de l'artilleur), quel est le nombre de cartes qu'il faut intercaler pour assurer la non-détonation de l'explosif (3 tirs négatifs). Le résultat du tir est apprécié à l'aide de la plaque témoin ; le résultat est positif si la plaque est percée d'un trou circulaire, négatif dans le cas contraire.

Les épreuves de sensibilité à l'impact au mouton Julius PETERS et de sensibilité au frottement Julius

4

PETERS sont décrites dans l'ouvrage de J. CALZIA, « Les substances explosives et leurs nuisances », pages 33 à 36.

L'épreuve de sensibilité au mouton de 30 kg consiste à déterminer le comportement pyrotechnique de 100 g de l'explosif soumis à l'impact d'une masse de 30 kg tombant d'une hauteur variable de 0 à 4 m.

On utilise un mouton à tête tronconique de 30 kg que l'on laisse chuter sur une extrémité d'une éprouvette en tôle de 8/10° de mm, de 50 × 8 × L mm, remplie d'explosif à sa densité gravimétrique. La longueur L est calculée de façon à avoir 100 g de produit.

On recherche la hauteur de chute pour laquelle on obtient 3 essais consécutifs négatifs, étant entendu qu'à la hauteur immédiatement supérieure (pas de 0,25 mm), il y a eu au moins un essai positif. L'essai positif est caractérisé par une explosion (déflagration ou détonation) avec au moins un début net de propagation le long de l'éprouvette (L/2 mm à compter du centre de l'impact).

L'épreuve nécessite 0,3 kg d'explosif.

L'épreuve dite de chauffage externe consiste à déterminer le confinement le plus faible (lumière calibrée) qui entraîne encore la réaction violente de l'explosif lorsque celui-ci est soumis à un chauffage extérieur.

L'explosif est chargé sur une hauteur de 60 mm dans une douille. La douille est en tôle d'acier emboutie de 0,5 mm d'épaisseur, et de dimensions : diamètre intérieur 24 mm, longueur 75 mm. Elle est fermée par une lumière calibrée qui est maintenue par un anneau fileté extérieurement et par un écrou coiffant vissé sur cet anneau. Le chauffage de la douille se fait grâce à 4 brûleurs au gaz propane qui délivrent une puissance d'environ 2 700 kcal/h.

On recherche le plus grand diamètre de lumière calibrée (en mm) pour laquelle on obtient une explosion de la douille en trois éclats au moins, on note :
— le temps T1 qui s'écoule entre l'allumage et l'inflammation,
— le temps T2 qui s'écoule entre l'allumage et l'explosion.

L'épreuve de combustion en gouttière consiste à déterminer la vitesse de propagation de flamme d'un filet d'explosif disposé à l'air libre dans une gouttière hémicylindrique de 20 mm de diamètre et 2 m de long et enflammé à son extrémité.

L'explosif est versé dans la gouttière disposée horizontalement, l'explosif étant maintenu en place par un bourrage de glaise à chaque extrémité.

On présente ensuite à une extrémité de la gouttière une source de chaleur (brandon imbibé d'alcool).

Cette épreuve permet en général d'apprécier d'une part, la facilité avec laquelle un produit peut s'enflammer à l'air libre, d'autre part, la forme et la vitesse de la réaction sous le conditionnement considéré.

Le résultat sur la vitesse est la moyenne d'au moins deux essais.

Exemple 6

On a déterminé les points de congélation de solutions placées dans des conditions de stockage. Les résultats obtenus sont regroupés dans le tableau suivant. Les compositions EX6-1 et EX6-2 ont été obtenues par dissolution respectivement de 45 et 60 g de T.N.T. dans 100 ml d'oléum sulfurique à 17 % en poids de $SO_3$ (de masse volumique 1,905 g/cm³).

| Composition | T.N.T. % en poids | $H_2SO_4$ % en poids | $SO_3$ % en poids | Température congélation |
|---|---|---|---|---|
| EX 2 | 18,9 | 60,8 | 20,3 | − 16 °C |
| EX 3 | 13,4 | 64,9 | 21,7 | − 30 °C |
| EX 4 | 23,7 | 57,2 | 19,1 | − 10 °C |
| EX 6-1 | 19,1 | 60,7 | 20,2 | + 6 °C |
| EX 6-2 | 24,0 | 57,0 | 19,0 | + 15 °C |

On voit que les compositions EX2, EX3 et EX4, qui font partie du domaine particulièrement préféré selon l'invention car elles contiennent de l'oléum à plus de 20 % d'anhydride sulfurique, présentent une bonne aptitude au stockage ou au transport dans des conditions de basse température.

**0 025 733**

Tous les essais ont été effectués en ensemençant les compositions à l'aide de germes de cristallisation constitués par de petits cristaux de T.N.T.

Si on ne procède pas ainsi, la température de congélation est fortement abaissée puisqu'elle devient, par exemple, de − 10 °C pour les compositions EX6-1 et EX6-2.

**Revendications**

1. Procédé de purification du trinitro-2,4,6 toluène, caractérisé en ce qu'on forme une solution homogène comprenant de 7 à 30 % en poids de trinitro-2,4,6 toluène à purifier, de 50 à 80 % en poids d'acide sulfurique et de 10 à 28 % en poids d'anhydride sulfurique, puis en ce qu'on provoque la séparation du trinitro-2,4,6 toluène et en ce qu'on recueille ce dernier à l'état solide sous une forme purifiée.

2. Procédé de purification du trinitro-2,4,6, toluène, selon la revendication 1, caractérisé en ce que la solution homogène comprend de 11 à 24 % en poids de trinitro-2,4,6 toluène, de 55 à 72 % en poids d'acide sulfurique et de 11 à 23 % en poids d'anhydride sulfurique.

3. Procédé selon l'une quelconque des revendications 1 et 2, caractérisé en ce qu'on provoque la séparation du trinitro-2,4,6 toluène par addition dans ladite solution homogène d'un fluide diminuant la solubilité du trinitro-2,4,6 toluène dans ladite solution.

4. Procédé selon la revendication 3, caractérisé en ce que le fluide est choisi dans le groupe constitué par l'eau et l'acide sulfurique faiblement concentré.

5. Procédé selon l'une quelconque des revendications 1 à 4, caractérisé en ce qu'on sépare le trinitro-2,4,6 toluène en abaissant la température de ladite solution jusqu'à précipitation du trinitro-2,4,6 toluène.

**Claims**

1. Process for the purification of 2,4,6-trinitrotoluene, characterised in that a homogeneous solution is formed comprising from 7 to 30 % by weight of 2,4,6-trinitrotoluene to be purified, from 50 to 80 % by weight of sulfuric acid and from 10 to 28 % by weight of sulfuric anhydride, then the separation of the 2,4,6-trinitrotoluene is effected and the 2,4,6-trinitrotoluene is recovered in purified form in the solid state.

2. Process for the purification of 2,4,6-trinitrotoluene according to claim 1, characterised in that the homogeneous solution comprises from 11 to 24 % by weight of 2,4,6-trinitrotoluene, from 55 to 72 % by weight of sulfuric acid and from 11 to 23 % by weight of sulfuric anhydride.

3. Process according to any one of claims 1 and 2, characterised in that the separation of the 2,4,6-trinitrotoluene is effected by the addition to said homogeneous solution of a fluid which reduces the solubility of 2,4,6-trinitrotoluene in said solution.

4. Process according to claim 3, characterised in that the fluid is chosen from the group consisting of water and weakly concentrated sulfuric acid.

5. Process according to any one of claims 1 to 4, characterised in that the 2,4,6-trinitrotoluene is separated by lowering the temperature of the said solution until the 2,4,6-trinitrotoluene precipitates.

**Ansprüche**

1. Verfahren zur Reinigung von 2,4,6-Trinitrotoluol, gekennzeichnet durch Herstellung einer homogenen Lösung, die 7 bis 30 Gew.-% zu reinigendes 2,4,6-Trinitrotoluol, 50 bis 80 Gew.-% Schwefelsäure und 10 bis 28 Gew.-% $SO_3$ enthält, Ausscheidung des 2,4,6-Trinitrotoluols und Gewinnung des gereinigten 2,4,6-Trinitrotoluols in festem Zustand.

2. Verfahren nach Anspruch 1, dadurch gekennzeichnet, daß die homogene Lösung 11 bis 24 Gew.-% 2,4,6-Trinitrotoluol, 55 bis 72 Gew.-% Schwefelsäure und 11 bis 23 Gew.-% $SO_3$ enthält.

3. Verfahren nach Anspruch 1 oder 2, dadurch gekennzeichnet, daß die Ausscheidung des 2,4,6-Trinitrotoluols durch Zugabe einer Flüssigkeit zu der homogenen Lösung hervorgerufen wird, die die Löslichkeit des 2,4,6-Trinitrotoluols darin verringert.

4. Verfahren nach Anspruch 3, dadurch gekennzeichnet, daß die Flüssigkeit unter Wasser und Schwefelsäure niedriger Konzentration ausgewählt wird.

5. Verfahren nach einem der Ansprüche 1 bis 4, dadurch gekennzeichnet, daß die Ausscheidung des 2,4,6-Trinitrotoluols durch Absenkung der Temperatur der Lösung bis zur Ausfällung des 2,4,6-Trinitrotoluols vorgenommen wird.